# DEMANDE DE BREVET EUROPEEN

(11) **EP 1 302 115 A1**
(43) Date de publication de la demande: **16.04.2003**
(21) Numéro de dépôt: 01203902.0
(22) Date de dépôt: 16.10.2001
(51) Int. Cl.: A23L 1/305

(54) **Utilisation de cystathionine**

(71) Demandeur: SOCIETE DES PRODUITS NESTLE S.A., 1800 Vevey (CH)
(72) Inventeur: Finot, Paul-André, 1806 St-Legier (FR); Huynh-Ba, Tuong, 1009 Pully (CH); Vuichoud, Jacques, 1814 La Tour-de-Peilz (CH); Breuille, Denis, 63450 Saint-Saturnin (FR)
(74) Mandataire: Vuille, Roman

(57) **Abrégé**

La présente invention concerne l'utilisation de cystathionine dans une composition nutritionnelle, un supplément alimentaire, une composition prophylactique, ou une formule infantile.

## Description

### DOMAINE DE L'INVENTION

La présente invention concerne une composition nutritionnelle, un supplément alimentaire, une composition prophylactique, et une formule infantile contenant de la cystathionine.

La demande de brevet EP 0655244 A1 décrit une composition nutritionnelle à base d'acides aminés pour le traitement d'infections, contenant une proportion de cystéine pharmacologiquement active supérieure à la proportion de cystéine correspondant aux besoins d'un homme sain. Cependant, la cystéine libre présente des inconvénients majeurs : d'une part elle résiste mal aux traitements thermiques puisqu'elle s'oxyde, notamment en cystine ; d'autre part, la cystéine a de mauvaises qualités organoleptiques car en dégageant faiblement de l'acide sulfhydrique et d'autres composés soufrés elle engendre une odeur ainsi qu'un goût très désagréable dans la solution nutritionnelle.

Considérant ces difficultés d'enrichir un produit en cystéine du fait de son instabilité, d'autres solutions doivent être trouvées pour enrichir les produits de nutrition, par exemple en utilisant des précurseurs de cystéine. La méthionine est un acide aminé indispensable qui permet la biosynthèse de cystéine en une succession de 5 étapes, passant par la synthèse d'homocystéine (en 3 étapes) puis par celle de cystathionine en une étape qui est irréversible puis finalement en cystéine. Il a été cependant montré que la fourniture de methionine ne permettait pas d'induire les effets bénéfiques observés par un enrichissement de la diète en cystéine. La présente invention a donc pour objet de proposer un moyen de surmonter cet obstacle et qui consiste à fournir de la cystathionine au titre de précurseur de cystéine; d'autre part il a été montré que la cystathionine était beaucoup plus stable que la cystéine.

La cystéine est l'un des 20 acides aminés que l'on trouve dans le corps humain. C'est un acide aminé non indispensable, ce qui signifie que l'organisme humain est capable de le synthétiser. Cependant, elle est importante car elle peut être transformée en de nombreux composés : la cystéine est oxydée en acide cystéine sulfinique, puis par décarboxylation en hypotaurine qui donne la taurine. Par décarboxylation également, elle peut aboutir à la synthèse d'acétyl CoA, et peut d'autre part être transformée en pyruvate. En tant qu'acide aminé soufré, la cystéine permet la formation de liaisons disulfures qui servent à stabiliser les structures tridimensionnelles des protéines extracellulaires circulantes, intracellulaires et membranaires. De plus, l'apport en cystéine est important dans les cas où l'on observe un stress de l'organisme, car elle est retrouvée en proportion importante dans les protéines synthétisées en réponse aux situations d'inflammation (qu'elle soit aiguë ou chronique), et est l'un des constituants du glutathion.

Le glutathion est un tripeptide composé de glycine, d'acide glutamique et de cystéine. Il agit comme coenzyme dans les cellules vivantes, intervient dans les réactions d'oxydoréduction et dans le transport des acides aminés. C'est aussi et surtout un des antioxydants les plus importants de l'organisme qui neutralise les radicaux libres et prévient ainsi les risques de cancers et de vieillissement. Enfin, le glutathion est un détoxiquant majeur du foie et participe ainsi à l'élimination des xénobiotiques.

Des études scientifiques ont été menées sur la cystathionine, in vitro, sur des cellules isolées, et in vivo. Dès 1942, l'article de Vigneaud et al. (J. Biol. Chem. , 143, 59, 1942) mentionne la possibilité que la cystathionine soit métabolisée en cystéine dans des tissus de mammifères. Depuis l'article de Hope (Proc. Intern. Congr. Biochem. 4^{th}, Vienna, 1958, 13, 63, 1960) il est bien connu que la cystathionine est une molécule intermédiaire dans la trans-sulfuration, qui transfère naturellement le soufre de la méthionine en passant par l'homocystéine pour donner la cystéine. Beatty P. W. et Reed D.J. (Arch. Biochem. Biophys., 1980, 204(1), 80-87) mentionnent quant à eux l'implication de la cystathionine dans la biosynthèse du glutathion. Enfin, très récemment l'article de MacCoss et al (Am. J. Physiol. Endocrinol. Metab, 2001, 280, E947-E955) mesure l'enrichissement plasmatique de la cystathionine après avoir perfusé de la méthionine marquée, quantifiant ainsi chez l'homme la vitesse de transformation de la méthionine en cystathionine.

La présente invention se propose de contrevenir à ces désagréments en enrichissant de compositions nutritionnelles en cystathionine, pour fournir de la cystéine et maintenir le statut en glutathion dans le corps du consommateur ou du patient.

### RESUME DE L'INVENTION

La présente invention concerne une composition nutritionnelle comprenant de la cystathionine pour la nutrition orale, enterale ou parenterale, un supplément alimentaire comprenant de la cystathionine, une composition prophylactique comprenant de la cystathionine, ou une formule infantile comprenant de la cystathionine.

### DESCRIPTION DE L'INVENTION

Comme mentionné ci-dessus, la cystéine présente des inconvénients majeurs, tant du point de vue de sa stabilité à la chaleur que du point de vue organoleptique. De manière surprenante, la cystahionine ne présente pas ces aspects négatifs. La cystathionine est un produit de condensation entre l'homocystéine et la sérine, et elle est convertie en cystéine par l'action de la cystathionase. Ce depside d'intérêt est naturellement présent dans les tissus de mammifères.

Un premier aspect de l'invention concerne l'utilisation de la cystathionine dans une composition nutritionnelle administrée par voie orale, entérale ou parentérale. Un second aspect de l'invention concerne les suppléments alimentaires contenant de la cystathionine. Un troisième aspect de l'invention concerne les compositions prophylactiques contenant de la cystathionine. Un quatrième aspect de l'invention concerne les formules infantiles contenant de la cystathionine. Un dernier aspect de l'invention concerne l'utilisation de la cystathionine associée à des fibres.

Par cystathionine, on entend la cystationine sous forme L, la cystathionine sous forme D, les sels de cystathionine ou un mélange de différentes formes de cystathionine.

Par composition nutritionnelle, on entend toute composition pour la nutrition orale, entérale, parentérale, culinaire ou alimentaire, entre autres, à destination de l'homme ou de l'animal. Par produit de nutrition entérale, on entend tout type de composition pouvant être introduite de façon artificielle dans l'organisme par la voie digestive. Par produit de nutrition parentérale, on entend toute composition (médicamenteuse ou nutritive, par exemple) pouvant être introduite dans l'organisme par une voie autre que la voie digestive: notamment par injection sous cutanée ou intramusculaire, par injection péritonéale, ou par perfusion intraveineuse, entre autres. Par composition alimentaire, on entend toute composition liquide, visqueuse, structurée, gélatineuse, solide, glacée, réfrigérée, en poudre, ou toute composition ayant subi une transformation manufacturée, notamment. La composition nutritionnelle selon la présente invention peut être conservable à température ambiante, réfrigérée, congelée, déshydratée; elle peut être, notamment, des liquides, des boissons, de la soupe, des mousselines ou purées, des céréales, de la mayonnaise, de la sauce à salade, de la pâte à tarte ou à pizza, des pâtes alimentaires, des surgelés, des crèmes desserts, des produits laitiers, ou encore de la poudre à café, du cacao en poudre, entre autres.

Par supplément alimentaire, on entend tout apport de nutriments destiné à améliorer la qualité du régime alimentaire d'une population donnée, saine ou non, humaine ou animale, et tout additif alimentaire. Cette définition comprend également toute substance ajoutée intentionnellement à un aliment, généralement en petites quantités, pour en faciliter la conservation, pour stabiliser ou améliorer ses qualités, sa texture, sa saveur, son aspect ou pour faciliter sa préparation. Par additif alimentaire, on entend toute substance dont l'emploi produit ou peut vraisemblablement produire comme résultat que ladite substance ou ses sous-produits deviennent partie intégrante d'un aliment ou modifient les caractères propres de cet aliment.

Le supplément alimentaire tel que défini dans la présente invention peut être sous forme de poudre, de gel, de liquide, d'émulsion, de tablettes ou toute autre forme ingérable avec ou sans liquide.
Par exemple, la cystathionine selon la présente invention pourrait être incorporée à un supplément alimentaire comprenant en outre des vitamines et/ou des minéraux.

Par composition prophylactique, on entend tout moyen destiné à prévenir l'apparition des maladies, ainsi que tout agent utilisé à titre protecteur ou préventif contre la maladie. La composition prophylactique selon la présente invention peut être utilisée chez l'homme ou l'animal, ce dernier pouvant être élevé de manière industrielle ou artisanale. Elle concerne, en particulier, les élevages de volailles, de lapins, de moutons, de porcs, de veaux, de rongeurs, ou encore de poissons.

La composition prophylactique selon la présente invention peut, outre la cystathionine, contenir d'autres acides aminés, par exemple. Elle peut être une composition nutritionnelle complète, un supplément alimentaire ou un produit injectable.

Par formule infantile, on entend tout substitut du lait maternel s'adressant à un enfant de moins de 12 mois couvrant les besoins nutritionnels normaux, ainsi que tout standard modifiable pour les enfants ayant des besoins nutritionnels spécifiques, et les formules de suite. Par formule de suite, on entend tout aliment constituent une partie de la diète de sevrage de l'âge de 6 mois à l'âge de 36 mois.

La teneur en cystathionine dans la composition nutritionnelle selon l'un des aspects de l'invention est de 0.01 à 100 g par litre ou par kilo de composition alimentaire. De préférence, la composition selon l'invention contient entre 0.1 et 50 g de cystathionine par litre ou 0.01 à 150 g par kilo de composition alimentaire.

Le reste de la composition peut contenir tout type d'ingrédients et d'excipients, notamment des sources de protéines (protéines entières, peptides et/ou acides aminés libres), des sources de lipides, notamment des acides gras modifiant la réponse inflammatoire, des sources de carbohydrates, des sources de fibres solubles et/ou insolubles, des vitamines (notamment des vitamines anti-oxydantes), des micronutriments, des colorants, des arômes, des stabilisants etc... Elle peut également contenir des facteurs de croissance, des bactéries et/ou leurs métabolites, des flavonoïdes ou des caroténoïdes dont le lycopène ou la lutéine, par exemple. Elle peut enfin contenir des molécules pharmaceutiques visant à agir contre tout type de maladie ou de syndrome, notamment le stress oxydatif, l'hypermétabolisme, le cancer ou les maladies cardio-vasculaires, par exemple.

La teneur en cystathionine dans le supplément alimentaire selon un second aspect de l'invention est comprise entre 0.1 et 100% dudit supplément (pourcentage en poids de matière sèche), et d'une manière encore plus optimale entre 1 et 50% en poids. Le reste du supplément peut contenir tout type d'excipient utilisable dans l'industrie agro-alimentaire, notamment des carbohydrates, protéines, arômes, ou colorants. Il peut également contenir d'autres suppléments alimentaires tels que des vitamines, acides aminés libres ou peptides, molécules pharmaceutiques, acidifiants, épaississants, agents sucrants ou édulcorants, antioxydants, lait ou protéines de lait, jus de fruits ou de légumes, arômes par exemple.

La teneur en cystathionine dans la composition prophylactique selon un troisième aspect de l'invention est comprise entre 0.1 et 99% de ladite composition (pourcentage en poids de matière sèche), et d'une manière encore plus optimale entre 30 et 60% en poids. Le reste de la composition peut contenir tout type de nutriment et d'excipient utilisable dans l'industrie agro-alimentaire, notamment des sucres, protéines, arômes, ou colorants. Elle peut également contenir d'autres suppléments alimentaires tels que des vitamines, acides aminés, antibiotiques, enzymes ou molécules pharmaceutiques, par exemple.

La teneur en cystathionine dans la formule infantile selon un quatrième aspect de l'invention est comprise entre 0.1 et 99% de ladite formule (pourcentage en poids de matière sèche), et d'une manière encore plus optimale entre 0.1 et 25% en poids. Le reste de la formule peut contenir tout type de nutriment ou d'aliment généralement utilisé dans l'industrie agro-alimentaire et particulièrement dans l'industrie alimentaire du nourrisson, notamment des carbohydrates, protéines, arômes, ou colorants. Il peut également contenir d'autres suppléments alimentaires tels que des vitamines, acides aminés libres ou peptides, molécules pharmaceutiques, acidifiants, épaississants, agents sucrants ou édulcorants, antioxydants, lait ou protéines de lait, jus de fruits ou de légumes, arômes par exemple.

L'utilisation de la cystathionine selon l'invention a pour but de fournir de la cystéine, pour maintenir le statut en glutathion et pour permettre la synthèse des protéines riches en cystéine intervenant dans certaines pathologies. Elle peut aussi être utilisée dans la nutrition du sportif pour préparer l'effort, pendant l'effort, mais aussi pendant la phase de récupération qui suit l'effort.
L'utilisation de la cystathionine pour faciliter la synthèse endogène du glutathion peut également intervenir dans les situations de stress oxydatif, d'hypermétabolisme, d'infection ou d'inflammation, de stress respiratoire ou de perte musculaire. Elle peut également être additionnée au régime alimentaire de personnes brûlées ou ayant des plaies importantes, d'enfants prématurés ou ayant un retard de croissance.

La cystathionine contenue dans une composition selon l'un quelconque des aspects de l'invention peut être associée à des hydrocolloïdes et à des fibres, par exemple, ce qui permet de pallier sa faible solubilité. Les fibres associées peuvent être, notamment, des gommes arabiques, des xanthanes, ou des gommes d'accacia. D'autres moyens pour augmenter la solubilité de la cystathionine ou pour augmenter la viscosité de la solution contenant la cystathionine peuvent être utilisés, même s'ils ne sont en rien indispensables pour la réalisation de la présente invention. Il peut s'agir, entre autres, de moyens tels que l'encapsulation ou l'utilisation d'émulsions doubles.

### ESSAIS SUR LES COMPORTEMENTS PHYSICO-CHIMIQUES ET ORGANOLEPTIQUES

La cystathionine se révèle être un depside très stable. Elle a été testée additionnée au SONDALIS (marque déposée), et comparée à la cystéine en concentration molaire équivalente (41 mM, soit 5g de cystéine /L). Le SONDALIS est un produit de nutrition entérale comprenant 35% de l'énergie sous forme de lipides, 50% sous forme de carbohydrates, notamment de maltodextrines, 15% sous forme de peptides, ainsi que des vitamines dont les vitamines A, B6, B12, C, D, E, K, et des sels minéraux tels que phosphore et magnésium, entre autres. 1.0 mL de SONDALIS additionné a été poussé à 121°C pendant 15 minutes dans un autoclave. Ceci représente un traitement thermique fort, la procédure ayant duré plus de deux heures depuis le début de la manipulation jusqu'à la phase de diminution de température. Ces conditions dures sont confirmées par une consistance épaisse et une couleur brune obtenue sur le produit.
Les résultats comparatifs figurent dans le tableau suivant.

| Solution testée | Odeur additionnelle |
|---|---|
| SONDALIS seul | Sans odeur additionnelle |
| SONDALIS + L cystathionine | Sans odeur additionnelle |
| SONDALIS + L cystéine | Odeur additionnelle soufrée |
| REABILAN seul | Sans odeur additionnelle |
| REABILAN + L cystathionine | Sans odeur additionnelle |
| REABILAN + L cystéine | Odeur additionnelle soufrée |

L'absence d'odeur dans le produit supplémenté en cystathionine est un premier élément important prouvant sa compatibilité avec une formule buvable, notamment, dans la mesure où l'oxydation et la décomposition des composés contenant du soufre produisent des dérivés ayant une forte odeur désagréable.

### ESSAIS SUR LA STABILITE

La stabilité de la cystathionine comparée à celle de la cystéine a été testée avec de l'eau désionisée et dans différentes formules entérales liquides (SONDALIS et REABILAN). La cystathionine a également été testée additionnée au REABILAN (marque déposée), qui est une composition entérale différente comprenant 35% de l'énergie sous forme de lipides, 52.5% sous forme de carbohydrates, notamment de maltodextrines et d'amidons, 12.5% sous forme de peptides, et comprenant également des vitamines, des sels minéraux, et d'autres composés tels que la taurine ou la L-carnitine. Les conditions de ce test ont été comparables à celles utilisées lors du test avec la composition SONDALIS.

La cystathionine a été ajoutée à son taux de solubilité maximale à 25°C, soit 0.6 mg/mL d'eau. Le mélange a été traité à 121°C pendant 15 minutes dans un autoclave et a été analysé.
Dans l'eau, la cystathionine est très stable même après un traitement à 121°C durant 15 minutes puisque le taux de récupération est de 100%, et la cystéine est légèrement oxydée en cystine (6%).
Dans les formules entérales, et avant traitement thermique, la cystéine est peu stable : elle est fortement oxydée et peut même être détruite, suivant la quantité ajoutée notamment. Contrairement à la cystéine, la cystathionine se révèle être très stable.
Dans les formules entérales, et après traitement thermique, le taux de cystéine disponible décroît de manière très importante, et la perte relative de cystéine augmente lorsque la quantité ajoutée diminue. La stabilité de la cystéine et celle de la cystathionine dépendent du produit dans lequel elles sont ajoutées ; cependant, et quelles que soient les conditions de manipulation testées, la cystathionine est toujours plus stable que la cystéine. Les résultats obtenus après traitement thermique (121°C pendant 15 minutes dans un autoclave) sont révélés dans le tableau suivant :

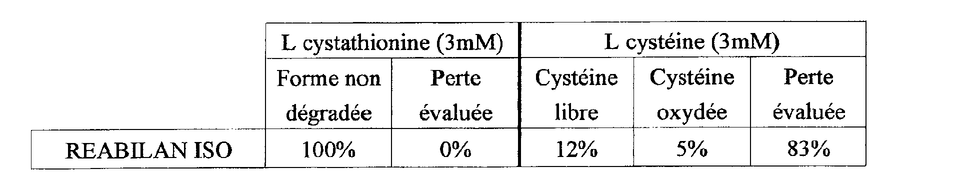

Contrairement à la cystéine, lorsque la cystathionine est ajoutée à une solution entérale, il n'y a pas de libération de H2S puisqu'il n'y a pas de libération d'odeur soufrée. La cystathionine présente donc des qualités organoleptiques très largement supérieures à celles de la cystéine ; d'autre part, 1a cystathionine est beaucoup plus stable à la chaleur que la cystéine.

Les exemples suivants sont délivrés uniquement à titre d'illustration.

### EXEMPLES

### EXEMPLE 1 :

On prépare un produit de nutrition entérale d'une valeur énergétique de 1000 kcal/L contenant en phase aqueuse 38g/L de protéines constituées par un mélange de protéines de caséine et de soja, 35 g/L d'un mélange de lipides comprenant des triglycérides à chaîne moyenne, de l'huile de maïs, de l'huile de soja, et de l'huile de colza, 125 g/L de carbohydrates sous forme de maltodextrines. Elle comprend en outre les composés cités dans le tableau suivant (où le mot "vitamine" est abrégé en "Vita", "ER" signifie "équivalent rétinol" et "ET" signifie "équivalent tocophérol)
Les protéines contiennent en outre 10 g/L de cystathionine.

| composé | unité | valeur | composé | unité | valeur |
|---|---|---|---|---|---|
| Vita A | µg/L ER | 450 | Vita B6 | mg/L | 1.4 |
| Vita E | mg/L ET | 10 | Acide panthoténique | | 5 |
| Vita D | µg/L | 2.5 | Choline | | 200 |
| Vita K | | 30 | Sodium | | 600 |
| Acide folique | | 180 | Potassium | | 1200 |
| Vita B12 | | 3 | Chlorure | | 1100 |
| Biotine | | 100 | Calcium | | 500 |
| Iode | | 100 | Phosphore | | 530 |
| Sélénium | | 44 | Magnésium | | 200 |
| Chrome | | 70 | Manganèse | | 2 |
| Molybdène | | 75 | Fer | | 10 |
| Vita C | mg/L | 53 | Cuivre | | 1 |
| Thiamine | | 1.0 | Zinc | | 10 |
| Riboflavine | | 1.2 | Fluorure | | 1 |
| Niacine | | 12 | | | |

### EXEMPLE 2 :

On prépare un supplément alimentaire destiné aux personnes âgées contenant 100 kcal pour 100 mL de supplément sous forme liquide. La composition finale du produite est la suivante:

| Composition nutritionnelle | |
|---|---|
| Protéines | 3.8 g |
| Carbohydrates | 13.8 g |
| Graisses | 3.3 g |
| Fibres | < 0.5 g |

| Minéraux | |
|---|---|
| Sodium | 0.035 g |
| Potassium | 130 mg |
| Calcium | 65 mg |
| Phosphore | 65 mg |
| Magnésium | 12 mg |
| Chlorure | 70 mg |
| Fer | 0.33 mg |
| Zinc | 0.75 mg |
| Cuivre | 0.10 mg |
| Manganèse | 0.20 mg |

| Vitamines | |
|---|---|
| Vitamine A | 80 µg (u.i) |
| Vitamine D | 0.50 µg (u.i) |
| Vitamine E | 2.0 mg |
| Vitamine K | 5.0 µg |
| Vitamine C | 10 mg |
| Vitamine B1 | 0.15mg |
| Vitamine B2 | 0.20 mg |
| Acide panthoténique | 0.63 mg |
| Vitamine B6 | 0.20 mg |
| Vitamine B12 | 0.50 µg |
| Niacine | 2.0 mg |
| Acide folique | 25 µg |
| Biotine | 0 015mg |

Les protéines utilisées dans cet exemple proviennent de lait écrémé en poudre, de petit lait, et de jaune d'oeuf en poudre.
Le supplément alimentaire décrit dans cet exemple contient 15 g de cystathionine.

### EXEMPLE 3 :

On prépare une formule infantile en mélangeant les ingrédients suivants dans les proportions indiquées. Le produit final contient 519 kcal pour 100 g, et se présente sous forme de poudre.

| | |
|---|---|
| Graisse | 27.7 g |
| Graisse de lait | 0.7 g |
| Mélange de graisses (150) | 26.8 g |
| Lécithine | 0.2 g |
| Acide linoléique | 4.1 g |
| Acide α-linolénique | 525 mg |
| Protéines | 9.5 g |
| Carbohydrates disponibles | 57.9 g |
| Lactose | 57.9 g |
| Minéraux (cendres) | 1.9 g |
| Sodium | 120 mg |
| Potassium | 460 mg |
| Chlorure | 330 mg |
| Calcium | 320 mg |
| Phosphore | 160 mg |
| Magnésium | 36 mg |
| Manganèse | 40 µg |
| Sélénium | 10.4 µg |
| Solides Totaux | 97.0 g |
| Humidité | 3.0 g |

Le profil en acides aminés des protéines présentes dans le produit est divulgué ci-après:

| Acide aminé | Quantité (mg/100kcal) | Acide aminé | Quantité (mg/100kcal) |
|---|---|---|---|
| Isoleucine | 104 | Valine | 106 |
| Leucine | 213 | Arginine | 81 |
| Lysine | 179 | Histidine | 45 |
| Méthionine | 45 | Alanine | 91 |
| Cystine | 21 | Acide aspartique | 199 |
| Cystathionine | 33 | Acide glutamique | 353 |
| Phénylalanine | 82 | Glycine | 48 |
| Tyrosine | 72 | Proline | 140 |
| Thréonine | 97 | Sérine | 95 |
| Tryptophane | 38 | Taurine | 8 |

La formule infantile comprend en outre les micronutriments communément utilisés dans les formules habituelles, dans les proportions habituelles.

## Revendications

1. Composition nutritionnelle comprenant de la cystathionine.

2. Composition nutritionnelle selon la revendication 1, **caractérisée** en que ladite composition est choisie dans le groupe constitué par un produit de nutrition entérale, un produit de nutrition parentérale, une composition alimentaire pour l'alimentation humaine, une composition alimentaire pour l'alimentation animale.

3. Composition nutritionnelle selon la revendication 1 ou la revendication 2, **caractérisée en ce que** la composition comprend entre 0.01 et 30g de cystathionine par litre ou par kilo de composition alimentaire.

4. Supplément alimentaire contenant de la cystathionine.

5. Supplément alimentaire selon la revendication 4 **caractérisé en ce que** ledit supplément contient entre 0.1 et 99% de cystathionine.

6. Supplément alimentaire selon la revendication 4 **caractérisé en ce que** ledit supplément contient entre 10 et 50% de cystathionine.

7. Composition prophylactique contenant de la cystathionine.

8. Composition prophylactique selon la revendication 7 **caractérisée en ce que** ladite composition contient entre 0.1 et 99% de cystathionine.

9. Composition prophylactique selon la revendication 7 **caractérisée en ce que** ladite composition contient entre 30 et 60% de cystathionine.

10. Formule infantile contenant de la cystathionine.

11. Formule infantile selon la revendication 10 **caractérisée en ce que** ladite formule contient entre 0.1 et 99% de cystathionine.

12. Formule infantile selon la revendication 10 **caractérisée en ce que** ladite formule contient entre 0.1 et 25% de cystathionine.

13. Utilisation de la cystathionine sous forme d'une composition selon l'une quelconque des revendications précédentes, ladite cystathionine étant associée à des fibres.
